# EUROPÄISCHE PATENTANMELDUNG

(11) **EP 4 446 421 A1**
(43) Veröffentlichungstag der Anmeldung: **16.10.2024**
(21) Anmeldenummer: 23167347.6
(22) Anmeldetag: 11.04.2023
(51) Int. Cl.: C12P 19/02

(54) **VERFAHREN ZUR HERSTELLUNG VON ALLITOL**

(71) Anmelder: Annikki GmbH, 8074 Raaba-Grambach (AT)
(72) Erfinder: Staunig, Nicole, 8074 Raaba-Grambach (AT); Dupont, Maria, 8074 Raaba-Grambach (AT)
(74) Vertreter: Schwarz & Partner Patentanwälte GmbH

(57) **Zusammenfassung**

Verfahren zur Herstellung von Allitol, indem aus D-Fructose, welche in einer wässerigen Lösung gelöst vorliegt, durch Behandeln mit einer Epimerase *in vitro* D-Psicose gebildet wird, welche durch Behandeln mit einer NAD(P)H-abhängigen Oxidoreduktase *in vitro* zu Allitol reduziert wird, wobei das durch die Reduktion entstandene NAD(P)' mit einem Wasserstoff-Donor enzymatisch wieder zu NAD(P)H reduziert wird, dadurch gekennzeichnet, dass als Wasserstoff-Donor ein sekundärer Alkohol eingesetzt wird. (Figur 1)

## Beschreibung

Die vorliegende Erfindung betrifft ein enzymatisches Verfahren zur Herstellung des seltenen Zuckeralkohols Allitol aus D-Fructose.

### Hintergrund der Erfindung

Allitol ist ein in der Natur selten vorkommender sechswertiger Zuckeralkohol, der beispielsweise in den Blättern von Rosmarinweiden (*Itea* sp.) nachgewiesen werden konnte (Hough & Stacey, 1963). Er ist achiral und bildet daher eine Schnittstelle zwischen den D- und L-Hexosen in der sogenannten Izumoring-Strategie (Izumori, 2006; Hassanin et al., 2017). Er kann daher als Vorstufe zur Herstellung von D-Psicose (Gullapalli et al., 2007; Poonperm et al., 2007) oder L-Psicose (Takeshita et al., 1996) dienen. Darüber hinaus kann Allitol aufgrund seines süßen Geschmacks auch als Süßungsmittel eingesetzt werden (Hassanin et al., 2017). WO 2020195106 A1 beschreibt eine Einsatzmöglichkeit von Allitol als Antiadipositum (Schlankheitsmittel).

Allitol ist von D-Fructose aus in zwei chemischen Umsetzungsschritten erreichbar: 1) Epimerisierung von D-Fructose zu D-Psicose (C3-Epimer) und 2) Reduktion von D-Psicose zu Allitol.

Der erste Schritt wird mit einer Ketose-3-Epimerase bewerkstelligt (Izumori et al., 1993). Ketose-3-Epimerasen lassen sich je nach Substratspezifität in drei Gruppen einteilen: 1) D-Tagatose-3-Epimerase (DTE), 2) D-Psicose-3-Epimerase (DPE) oder D-Allulose-3-Epimerase (DAE) und 3) L-Ribulose-3-Epimerase (LRE). Bei der Epimerisierung bildet sich ein Gleichgewichtsverhältnis zwischen beiden Epimeren aus. Je nach Reaktionsbedingungen (Temperatur zwischen 40 und 70 °C, pH-Wert zwischen 6 und 11) liegt dieses zwischen 80:20 und 62,5:37,5 (D-Fructose : D-Psicose). Viele der Epimerasen benötigen außerdem ein zweiwertiges Metallion wie Mn2⁺ oder Co²⁺ (toxisch) als Kofaktor (Zhang et al., 2016; Jiang et al., 2020).

Die Reduktion von D-Psicose zu Allitol kann z.B. mikrobiell mit *Enterobacter agglomerans* Stamm 221e (Muniruzzaman et al., 1995) oder mit *Klebsiella oxytoca* G4A4 (Han et al., 2014) durchgeführt werden.

Ribitol-Dehydrogenase (RDH; EC 1.1.1.56), die für die Umwandlung des fünfwertigen Zuckeralkohols Ribitol zur Ketopentose D-Ribulose verantwortlich ist, katalysiert ebenfalls die Reduktion von D-Psicose zu Allitol.

Hassanin et al. (2016) nutzten eine Ribitol-Dehydrogenase (RDH) aus *Providencia alcalifaciens* RIMD 1656011 und eine Formiat-Dehydrogenase (FDH) aus *Ogataea parapolymorpha* DL-1 (zur Regeneration des Kofaktors Nicotinamidadenindinukleotid NADH; oxidiert Formiat zu CO₂) in Form von Zell-Lysaten zum Umsatz von D-Psicose (10 g/l) zu Allitol (84% Umsatz in 6 h; Zusatz von 2 mM NAD⁺).

Zur Gewinnung von Allitol wurde eine HPLC-Methode verwendet - das gesammelte Eluat wurde gefriergetrocknet.

Zhu et al. (2015) exprimierten D-Psicose-3-Epimerase aus *Ruminococcus* sp. und RDH aus *Klebsiella oxytoca,* eine FDH aus *Candida methylica* sowie ein Glucose-Fructose-Facilitator-Genprodukt aus *Zymomonas mobilis* in *Escherichia coli.* Mit diesen Modifikationen konnte der rekombinante Stamm 16,5 g/l Allitol aus 18 g/l D-Fructose (92% Umsatz nach 18 h) produzieren.

Wen et al. (2022) nutzten ebenfalls einen rekombinanten *E. coli*-Stamm (mit exprimierter RDH und FDH), der 58,5 g/l Allitol aus 90 g/l D-Psicose in 1 h produzierte.

Zhao et al. (2022) verwendeten ein Multienzym-Selbstassemblierungssystem aus DPE, RDH und Glucose-Dehydrogenase (GDH; zur Kofaktor-Regeneration) in Kombination mit einer Glucose-Isomerase (GI) in einem Ganzzell-System, um Allitol (15 g/l) direkt aus D-Glucose (25 g/l) herzustellen. Ein großer Nachteil an der Verwendung einer GDH zur Regeneration des Redox-Kofaktors NAD(P)H ist die Bildung von Gluconolacton (Lacton der Gluconsäure) als Nebenprodukt, das unter Aufwand entfernt werden muss.

Wang et al. (2023) beschreiben einen E. coli-Ganzzell-Biokatalysator zur Umwandlung von D-Fructose zu Allitol. Die verwendeten *E. coli*-Zellen beinhalteten eine DPE aus *Clostridiales,* eine RDH aus *Providencia alcalifaciens,* eine FDH aus *Starkeya* sowie eine weitere DPE aus *Rhizobium straminoryzae.* Auf diese Weise wurde D-Fructose (500 mM = 90 g/l) innerhalb von 12 h bei 37 °C und pH 6 unter Einsatz von 1000 mM Natrium-Formiat (zwei Äquivalente bezogen auf D-Fructose) und 0,5 mM NAD⁺ zu 452 mM Allitol umwandelt (Umsatz 90,4%). Als Nebenprodukt entstand ca. 30 mM D-Sorbitol (das Reduktionsprodukt der D-Fructose). Die Zellen wurden durch Zentrifugation abgetrennt und ausgetretene Proteine im Allitol-haltigen Überstand wurden durch Hitzeeinwirkung deaktiviert.

Die Verwendung einer FDH zur Kofaktor-Regeneration weist einige Nachteile auf: 1. Bildung von klimaschädlichem CO₂ als Oxidationsprodukt von Formiat, 2. Verschiebung des pH-Werts der Reaktion in den basischen Bereich (Neuhauser et al., 1998; Kratzer et al., 2015), 3. Produktion großer Mengen an Abfall bestehend aus nicht umgesetztem Natrium-Formiat und Natriumsulfat (bei Verwendung von Schwefelsäure zur pH-Kontrolle) sowie 4. die geringe spezifische Aktivität von Formiat-Dehydrogenasen (Boldt & Ansorge-Schumacher, 2020; Tishkov & Popov, 2004).

Takeshita et al. (Journal of Bioscience and Bioengineering, 90(5), 545-548, 2000) verwendeten eine DTE aus *Pseudomonas cichorii* ST-24 und eine RDH aus *Klebsiella pneumoniae* Stamm X22 (eine Mutante von *K. pneumoniae* IFO 3321) in Form von Zell-Lysaten (zellfreien Extrakten) zur Umsetzung von D-Fructose (10 g/l) unter Zusatz von 2,5 mM NAD⁺ in 48 h zu Allitol (100% Umsatz). Zur Regeneration des Kofaktors wurde eine FDH verwendet. Reines Allitol wurde durch die Behandlung der Reaktionsmischung mit Aktivkohle, Zentrifugation/Filtration gefolgt von Deionisation mittels lonentauscherharzen aus der Produktlösung gewonnen. Ein Nachteil dieses Verfahrens besteht darin, dass selbst unter optimierten Bedingungen D-Fructose nur in relativ niedrigen Konzentrationen von etwa 10 g/l umgesetzt werden kann.

Hier setzt nun die Aufgabe der vorliegenden Erfindung an und setzt sich zum Ziel, ein Verfahren zur Herstellung von Allitol zur Verfügung zu stellen, welches das oben genannte Verfahren von Takeshita et al. verbessert und welches insbesondere gestattet, höhere D-Fructose-Konzentrationen einsetzen zu können.

### Detaillierte Beschreibung der Erfindung

Die Aufgabe wird erfindungsgemäß gelöst, indem aus D-Fructose, welche in einer wässerigen Lösung vorliegt, durch Behandeln mit einer Epimerase *in vitro* D-Psicose gebildet wird, die durch Behandeln mit einer NAD(P)H-abhängigen Oxidoreduktase *in vitro* zu Allitol reduziert wird, wobei das bei der Reduktion entstandene NAD(P)⁺ mit einem Wasserstoff-Donor enzymatisch wieder zu NAD(P)H reduziert wird, dadurch gekennzeichnet, dass als Wasserstoff-Donor ein sekundärer Alkohol eingesetzt wird.

Es hat sich gezeigt, dass bei erfindungsgemäßem Einsatz eines sekundären Alkohols als Wasserstoff-Donor - anstatt von Ameisensäure (Formiat) als Wasserstoff-Donor - D-Fructose in einer viel höheren Konzentration eingesetzt werden kann als im Stand der Technik.

Die erfindungsgemäß eingesetzte Regenerierung des Kofaktors ist z.B. aus der EP 2812439 B1 vorbekannt oder von Xu et al. (2021) beschrieben.

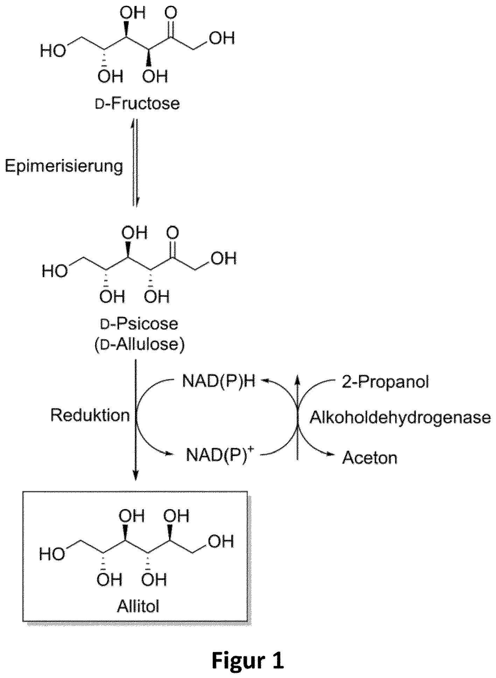

Im ersten Schritt wird aus D-Fructose durch Behandeln mit einer Epimerase *in vitro* D-Psicose gebildet, welches mit einer NAD(P)-abhängigen Oxidoreduktase *in vitro* zu Allitol reduziert wird. Allitol kann im Anschluss durch Kristallisation aus der Lösung abgetrennt werden. Das erfindungsgemäße Verfahren ist in der beiliegenden Figur 1 schematisch dargestellt.

Eine bevorzugte Variante des erfindungsgemäßen Verfahrens besteht darin, dass der sekundäre Alkohol 2-Propanol (Isopropanol) ist. 2-Propanol ist ein sehr günstiger Wasserstoff-Donor zur Regeneration von NAD(P)H und das Oxidationsprodukt Aceton ist aufgrund seiner Flüchtigkeit leicht abtrennbar (Xu et al., 2021). Aus dem Abgasstrom gewonnenes Aceton kann heterogen-katalytisch in der Gasphase zu wieder zu 2-Propanol hydriert werden (Al-Rabiah et al., 2022), wobei in Zukunft vermehrt auf Wasserstoff aus nachhaltigen Quellen ("grüner Wasserstoff") gesetzt werden könnte. Die Verwendung eines solchen Systems erlaubt das Recycling von 2-Propanol ohne die Emission von klimaschädlichem CO₂.

Eine weitere bevorzugte Variante des erfindungsgemäßen Verfahrens ist dadurch gekennzeichnet, dass es als Eintopf-Reaktion ohne Isolierung von Zwischenprodukten durchgeführt wird.

Die besonders bevorzugte Konzentration von D-Fructose beträgt 100 - 250 g/l.

Der besonders bevorzugte Temperaturbereich für das erfindungsgemäße Verfahren liegt zwischen 25 und 45 °C.

Der besonders bevorzugte pH-Bereich liegt zwischen 7 und 8,5.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme* & *Herstellung der Lysate* für Details).

In einer weiteren Variante können die Enzyme auch auf einem festen Trägermaterial immobilisiert sein.

Gemäß einem weiteren Aspekt der Erfindung finden alle Schritte in einem Reaktionsgefäß statt (*Eintopf-Reaktion*).

In einer besonders bevorzugten Ausführung des Verfahrens werden für die Konversion des Startmaterials nur Enzyme aus den Enzymgruppen Epimerasen, Dehydrogenasen und Reduktasen verwendet, wobei eines oder mehrere dieser Enzyme aus jeder dieser Gruppen ausgewählt werden.

Die im Verfahren verwendete Epimerase kann aus einer der Gruppen EC 5.1.3.30 (D-Psicose-3-Epimerase) oder EC 5.1.3.31 (D-Tagatose-3-Epimerase/L-Ribulose-3-Epimerase) stammen, wobei die erstgenannte besonders bevorzugt ist.

Das zur Reduktion von D-Psicose verwendete Enzym stammt aus der Gruppe der Oxidoreduktasen, wobei eine Ribitol-Dehydrogenase (EC 1.1.1.56) besonders bevorzugt ist.

Die zur Kofaktor-Regeneration eingesetzte Alkoholdehydrogenase (ADH) kann aus einer der Gruppen EC 1.1.1.1 (NAD-abhängige ADH) und EC 1.1.1.2 (NADP-abhängige ADH) stammen.

Die hier vorgestellte enzymatische Strategie in Kombination mit der Kofaktor-Regeneration ermöglicht einen biokatalytischen, umweltfreundlichen und hocheffizienten Produktionsprozess zur Herstellung von Allitol.

Im Vergleich zu dem im Stand der Technik beschriebenen Regenerationsverfahren mit FDH bietet die Kofaktor-Regeneration mit ADH noch weitere Vorteile: geringere Salzkonzentrationen (kein Einsatz von Natrium-Formiat), keine Emission von CO₂ sowie die Möglichkeit des Recyclings von 2-Propanol durch Reduktion von Aceton mit grünem Wasserstoff. Darüber hinaus können Substrat-Konzentrationen von ≥ 100 g/l durch die Anwendung der Kofaktor-Regeneration mit ADH umgesetzt werden.

### Materialien

D-Psicose wurde von TCI und Hunan Garden Naturals Inc. (China), Allitol wurde von TCI, D-Fructose, NADPH-Tetranatriumsalz, und Methanol wurden von PanReac AppliChem (ITW Reagents), Zinkchlorid, IPTG (Isopropyl-β-D-thiogalactopyranosid) wurden von Sigma-Aldrich, Magnesiumchlorid-Hexahydrat, Kaliumdihydrogenphosphat, di-Kaliumhydrogenphosphat, NAD⁺, NADH-Dinatriumsalz, NADP⁺-Dinatriumsalz sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E. coli*-Zellen Top10F transformiert und die entstandenen Kolonien werden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert. Die rekombinante Expression des Target-Enzymes erreicht meistens 50 % des *E. coli*-Gesamtproteins.

### Herstellung von Zell-Lysaten mittels GEA-Aufschlusses

Zur Herstellung einer Zell-Suspension (Volumen > 50 ml) wurde das nach obigen Verfahren hergestellte Zellpellet in einem geeigneten Gefäß eingewogen und in Puffer (Triethanolamin (TEA) - HCl oder Kaliumphosphat-Puffer (KPP); siehe Tabelle 1 für verwendete Puffer-Systeme) gelöst. Der Massenanteil an Biomasse beträgt üblicherweise 20%, der Rest entfällt auf den Puffer.

Zum Zell-Aufschluss wurde ein Lab Homogenizer PandaPLUS 2000 von GEA verwendet (Druck ca. 1000 bar).

Die Homogenate wurden 10 min lang bei 4 °C und 16000 rpm zentrifugiert (Hermle Z 446 K Zentrifuge), um die unlöslichen Zellfragmente abzutrennen und das Lysat zu erhalten.

**Tabelle 1. Enzymklassen, Spenderorganismen und Aufschlussbedingungen für die in den Beispielen verwendeten Enzyme.**

| **Enzymtyp (EC-Klasse)** | **katalysierte Reaktion** | **Spenderorganismus** | **Aufschlussbedingungen** | **Literatur** |
|---|---|---|---|---|
| D-Psicose-3-Epimerase (EC 5.1.3.30) | D-Fructose → D-Psicose | *Clostridium cellulolyticum* H10 | GEA (20% Biomasse in100 mM TEA-HCl pH 7 + 2 mM MgCl₂) | (Mu et al., 2011; Chan et al., 2012) |
| Ribitol-Dehydrogenase (EC 1.1.1.56) | D-Psicose → Allitol | *Klebsiella pneumoniae* | GEA (20% Biomasse in 100 mM TEA-HCl pH 7) | (Takeshita et al., 2000; Sequenz in NLM Protein Database: WP_265716617.1) |
| Alkoholdehydro genase (ADH) (EC 1.1.1.1) | 2-Propanol → Aceton | *(Geo-)Bacillus stearothermophilus* NCA1503 | GEA (20% Biomasse in 100 mM TEA-HCl pH 7 + 0,5 mM ZnCl₂) | (Sakoda & Imanaka, 1992) |

### Analytische Methoden

### High Performance Liquid Chromatography

Zur Quantifizierung von Allitol, D-Psicose und D-Fructose mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RPM-Monosaccharide Pb+2 (8%) Säule mit entsprechender Vorsäule verwendet und mit Reinstwasser isokratisch eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCI-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH bzw. NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit dem nachfolgenden Beispiel wird eine bevorzugte Variante des erfindungsgemäßen Verfahrens noch näher beschrieben. Die in diesen Beispielen eingesetzten Lysate wurden nach den oben beschriebenen Verfahren hergestellt.

### Beispiel

In einem 2 ml Glas-Vial wurden folgende Komponenten vermischt: 189,3 µl deionisiertes Wasser, 50 µl eines 500 mM TEA-HCl-Puffers (pH 8), 100 µl einer D-Fructose-Lösung (500 g/l) sowie 35 µl D-Psicose-3-Epimerase-Lysat. Anschließend wurden 50 µl Ribitol-Dehydrogenase-Lysat, 12 U Alkoholdehydrogenase-Lysat, 10 µl einer 10 mM NAD⁺-Lösung sowie 50 µl 2-Propanol zum Ansatz hinzugefügt. Der Ansatz wurde unter kontinuierlichem Schütteln (Eppendorf-Thermomixer, 35 °C, 1200 rpm) für insgesamt 56 h inkubiert.

Nach 24 und 48 h wurden jeweils 50 µl einer 2-Propanol/Wasser-Mischung (2/3 v/v) zum Ansatz hinzugefügt.

Zur Analyse wurden 100 µl des Ansatzes mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen.

Auf diese Weise wurden 94,2% der D-Fructose (100 g/l) zu Allitol (gefundene Konzentration: 83,0 g/l) umgesetzt.

Zur Kristallisation wurden 100 ml derselben Mischung verwendet, auf pH 4 angesäuert und bei 70 °C für 30 min gerührt. Die heiße Reaktionsmischung wurde durch eine Glasfritte (P4, 10-16 µm) mittels Anlegen eines Vakuums filtriert. Das Filtrat wurde zuerst auf Raumtemperatur abgekühlt und danach über Nacht im Kühlschrank bei 4 °C gelagert. Der farblose Niederschlag wurde abfiltriert (Glasfritte P4, 10-16 µm) und für 24 h im Vakuumtrockenschrank bei 50 °C getrocknet.

Das Filtrat wurde mit Aceton versetzt und über Nacht im Kühlschrank bei 4 °C gelagert. Der Niederschlag wurde abfiltriert (Glasfritte P4, 10-16 µm) und mit eiskaltem Aceton gewaschen. Das Produkt wurde für 24 h im Vakuumtrockenschrank bei 50 °C getrocknet. Eine HPLC-Analyse der Produktfraktionen ergab, dass es sich bei dem Produkt um Allitol handelte, welches in hoher Reinheit (≥ 99%) gewonnen werden konnte.

### Literatur

Hough, L., & Stacey, B. E. (1963). The occurrence of D-ribohexulose in Itea ilicifolia, Itea virginica, and Itea yunnanensis. Phytochemistry, 2(4), 315-320. https://doi.org/10.1016/S0031-9422(00)84854-2
Izumori, K. (2006). Izumoring: a strategy for bioproduction of all hexoses. Journal of Biotechnology, 124(4), 717-722. https://doi.org/10.1016/j.jbiotec.2006.04.016
Hassanin, H. A. M., Mu, W., Koko, M. Y. F., Zhang, T., Masamba, K., & Jiang, B. (2017). Allitol: production, properties and applications. International Journal of Food Science and Technology, 52(1), 91-97. https://doi.org/10.1111/ijfs.13290
Gullapalli, P., Takata, G., Poonperm, W., Rao, D., Morimoto, K., Akimitsu, K., Tajima, S., & Izumori, K. (2007). Bioproduction of D-psicose from allitol with Enterobacter aerogenes IK7: a new frontier in rare ketose production. Bioscience, Biotechnology, and Biochemistry, 71(12), 3048-3054. https://doi.org/10.1271/bbb.70450
Poonperm, W., Takata, G., Ando, Y., Sahachaisaree, V., Lumyong, P., Lumyong, S., & Izumori, K. (2007). Efficient conversion of allitol to D-psicose by Bacillus pallidus Y25. Journal of Bioscience and Bioengineering, 103(3), 282-285. https://doi.org/10.1263/jbb.103.282
Takeshita, K., Shimonishi, T., & Izumori, K. (1996). Production of L-psicose from allitol by Gluconobacter frateurii IFO 3254. Journal of Fermentation and Bioengineering, 81(3), 212-215. https://doi.org/10.1016/0922-338X(96)82210-0
Izumori, K., Khan, A. R., Okaya, H., & Tsumura, T. (1993). A New Enzyme, D-Ketohexose 3-Epimerase, from Pseudomonas sp. ST-24. Bioscience, Biotechnology, and Biochemistry, 57(6), 1037-1039. https://doi.org/10.1271/bbb.57.1037
Zhang, W., Yu, S., Zhang, T., Jiang, B., & Mu, W. (2016). Recent advances in D-allulose: Physiological functionalities, applications, and biological production. Trends in Food Science and Technology, 54, 127-137. https://doi.org/10.1016/j.tifs.2016.06.004
Jiang, S., Xiao, W., Zhu, X., Yang, P., Zheng, Z., Lu, S., Jiang, S., Zhang, G., & Liu, J. (2020). Review on D-Allulose: In vivo Metabolism, Catalytic Mechanism, Engineering Strain Construction, Bio-Production Technology. Frontiers in Bioengineering and Biotechnology, 8, 26. https://doi.org/10.3389/fbioe.2020.00026
Muniruzzaman, S., Tokunaga, H., & Izumori, K. (1995). Conversion of D-psicose to allitol by Enterobacter agglomerans strain 221e. Journal of Fermentation and Bioengineering, 79(4), 323-327. https://doi.org/10.1016/0922-338X(95)93989-W
Han, W., Zhu, Y., Men, Y., Yang, J., Liu, C., & Sun, Y. (2014). Production of allitol from D-psicose by a novel isolated strain of Klebsiella oxytoca G4A4. Journal of Basic Microbiology, 54(10), 1073-1079. https://doi.org/10.1002/jobm.201300647
Hassanin, H. A. M., Letsididi, R., Koko, M. Y. F., Mu, W., Elferga, A., & Jiang, B. (2016). Synthesis of allitol from D-psicose using ribitol dehydrogenase and formate dehydrogenase. Tropical Journal of Pharmaceutical Research, 15(12), 2701-2708. https://doi.org/10.4314/tjpr.v15i12.23
Zhu, Y., Li, H., Liu, P., Yang, J., Zhang, X., & Sun, Y. (2015). Construction of allitol synthesis pathway by multi-enzyme coexpression in Escherichia coli and its application in allitol production. Journal of Industrial Microbiology & Biotechnology, 42(5), 661-669. https://doi.org/10.1007/s10295-014-1578-1
Wen, X., Lin, H., Ren, Y., Li, C., Zhang, C., Lin, J., & Lin, J. (2022). Allitol bioproduction by recombinant Escherichia coli with NADH regeneration system co-expressing ribitol dehydrogenase (RDH) and formate dehydrogenase (FDH) in individual or in fusion. Electronic Journal of Biotechnology, 55, 91-98. https://doi.org/10.1016/j.ejbt.2021.11.007
Zhao, J., Guo, Y., Li, Q., Chen, J., Niu, D., & Liu, J. (2022). Reconstruction of a Cofactor Self-Sufficient Whole-Cell Biocatalyst System for Efficient Biosynthesis of Allitol from D-Glucose. Journal of Agricultural and Food Chemistry, 70(12), 3775-3784. https://doi.org/10.1021/acs.jafc.2c00440
Wang, L., Chen, K., Zheng, P., Huo, X., Liao, F., Zhu, L., Hu, M., & Tao, Y. (2023). Enhanced production of D-psicose from D-fructose by a redox-driven multi-enzyme cascade system. Enzyme and Microbial Technology, 163, 110172. https://doi.org/10.1016/j.enzmictec.2022.110172
Neuhauser, W., Steininger, M., Haltrich, D., Kulbe, K. D., & Nidetzky, B. (1998). A pH-Controlled Fed-Batch Process Can Overcome Inhibition by Formate in NADH-Dependent Enzymatic Reductions Using Formate Dehydrogenase-Catalyzed Coenzyme Regeneration. Biotechnology and Bioengineering, 60(3), 277-282. https://doi.org/10.1002/(SICI)1097-0290(19981105)60:3<277::AID-BIT2>3.0.CO;2-E
Kratzer, R., Woodley, J. M., & Nidetzky, B. (2015). Rules for biocatalyst and reaction engineering to implement effective, NAD(P)H-dependent, whole cell bioreductions. Biotechnology Advances, 33(8), 1641-1652. https://doi.org/10.1016/j.biotechadv.2015.08.006
Boldt, A., & Ansorge-Schumacher, M. B. (2020). Formate Dehydrogenase from Rhodococcus jostii (RjFDH) - A High-Performance Tool for NADH Regeneration. Advanced Synthesis und Catalysis, 362(19), 4109-4118. https://doi.org/10.1002/adsc.202000536
Tishkov, V. I., & Popov, V. O. (2004). Catalytic Mechanism and Application of Formate Dehydrogenase. Biochemistry (Moscow), 69(11), 1252-1267. https://doi.org/10.1007/s10541-005-0071-x
Takeshita, K., Ishida, Y., Takada, G., & Izumori, K. (2000). Direct Production of Allitol from D-Fructose by a Coupling Reaction Using D-Tagatose 3-Epimerase, Ribitol Dehydrogenase and Formate Dehydrogenase. Journal of Bioscience and Bioengineering, 90(5), 545-548. https://doi.org/10.1016/51389-1723(01)80038-4
Xu, J., Zhou, H., Yu, H., Deng, T., Wang, Z., Zhang, H., Wu, J., & Yang, L. (2021). Computational design of highly stable and soluble alcohol dehydrogenase for NADPH regeneration. Bioresources and Bioprocessing, 8, 12. https://doi.org/10.1186/s40643-021-00362-w
Al-Rabiah, A. A., Boz, I., Akhmedov, V. M., Mostafa, M. M. M., & Bagabas, A. A. (2022). Highly Selective Gas-Phase Catalytic Hydrogenation of Acetone to Isopropyl Alcohol. Catalysts, 12(10), 1251. https://doi.org/10.3390/catal12101251
Lin, B., & Tao, Y. (2017). Whole-cell biocatalysts by design. Microbial Cell Factories, 16, 106. https://doi.org/10.1186/s12934-017-0724-7
Mu, W., Chu, F., Xing, Q., Yu, S., Zhou, L., & Jiang, B. (2011). Cloning, Expression, and Characterization of a D-Psicose 3-Epimerase from Clostridium cellulolyticum H10. Journal of Agricultural and Food Chemistry, 59(14), 7785-7792. https://doi.org/10.1021/jf201356q
Chan, H.-C., Zhu, Y., Hu, Y., Ko, T.-P., Huang, C.-H., Ren, F., Chen, C.-C., Ma, Y., Guo, R.-T., & Sun, Y. (2012). Crystal structures of D-psicose 3-epimerase from Clostridium cellulolyticum H10 and its complex with ketohexose sugars. Protein & Cell, 3(2), 123-131. https://doi.org/10.1007/s13238-012-2026-5
Protein [Internet]. Bethesda (MD): National Library of Medicine (US), National Center for Biotechnology Information. Accession No. WP_265716617.1, SDR family oxidoreductase [Klebsiella pneumoniae]. Verfügbar unter: https://www.ncbi.nlm.nih.gov/protein/WP_265716617.1 (Zugriff am 04.04.2023)
Sakoda, H., & Imanaka, T. (1992). Cloning and sequencing of the gene coding for alcohol dehydrogenase of Bacillus stearothermophilus and rational shift of the optimum pH. Journal of Bacteriology, 174(4), 1397-1402. https://doi.org/10.1128/jb.174.4.1397-1402.1992

## Patentansprüche

1. Verfahren zur Herstellung von Allitol, indem aus D-Fructose, welche in einer wässerigen Lösung vorliegt, durch Behandeln mit einer Epimerase *in vitro* D-Psicose gebildet wird, die durch Behandeln mit einer NAD(P)H-abhängigen Oxidoreduktase *in vitro* zu Allitol reduziert wird, wobei das bei der Reduktion entstandene NAD(P)⁺ mit einem Wasserstoff-Donor enzymatisch wieder zu NAD(P)H reduziert wird,
**dadurch gekennzeichnet,**
**dass** als Wasserstoff-Donor ein sekundärer Alkohol eingesetzt wird.

2. Verfahren nach Anspruch 1, **dadurch gekennzeichnet, dass** der sekundäre Alkohol 2-Propanol ist.

3. Verfahren nach einem der Ansprüche 1 bis 2, **dadurch gekennzeichnet, dass** es als Eintopf-Reaktion ohne Isolierung von Zwischenprodukten durchgeführt wird.

4. Verfahren nach einem der Ansprüche 1 bis 3, **dadurch gekennzeichnet, dass** die Enzyme als Lysat der entsprechenden, sie bildenden Zellen vorliegen.
